# EUROPÄISCHE PATENTANMELDUNG

(11) **EP 0 788 776 A1**
(43) Veröffentlichungstag der Anmeldung: **13.08.1997**
(21) Anmeldenummer: 97102013.6
(22) Anmeldetag: 07.02.1997
(51) Int. Cl.: A61B 19/00, A61G 10/00

(54) **Verfahren zur Vermeidung der Sprühkontamination bei medizinischen Eingriffen sowie in diesem verwendbare Vorrichtung**

(30) Priorität: 10.02.1996 DE 19604955
(71) Anmelder: Ostermann, Stefan, Dipl.-Ing., 51467 Bergisch Gladbach (DE); Luig, Rainer, Dipl.-Kaufmann, 58706 Menden (DE)
(72) Erfinder: Ostermann, Stefan, Dipl.-Ing., 51467 Bergisch Gladbach (DE); Luig, Rainer, Dipl.-Kaufmann, 58706 Menden (DE)
(74) Vertreter: Fritz, Edmund Lothar, Dipl.-Chem.

(57) **Zusammenfassung**

Die Erfindung betrifft ein Verfahren zur Vermeidung der Sprühkontamination bei medizinischen Eingriffen. Bei dem Verfahren wird um das zu behandelnde Körperteil (6) ein kleiner Behandlungsraum gebildet und Zugangsmöglichkeiten (41) in das Innere des Behandlungsraumes geschaffen. Die bei der Behandlung entstehenden verunreinigten festen, flüssigen und gasförmigen Stoffe werden durch die Begrenzung (1, 4) des Behandlungsraums nahezu vollständig von der Außenumgebung abgehalten. Die Stoffe, insbesondere die gasförmigen Stoffe werden aus dem Innern des Behandlungsraums mittels einer Absaug- und Filtereinrichtung (54) abgesaugt -und einer weiteren Behandlung (51, 52, 53) unterzogen. Durch die Anwendung des erfindungsgemäßen Verfahrens wird somit die Gefahr einer Sprühkontaminierung des Patienten selbst und auch der behandelnden Personen weitesgehend vermieden. Durch die Absaugung und Behandlung der Stoffe erhöht sich die Sicherheit aller Beteiligten.

## Beschreibung

In der medizinischen Behandlung werden z. B. bei chirurgischen Eingriffen die Lasertechnik, das Fräsen und Schleifen und in neuerer Zeit auch das Hochdruck-Wasserstrahlschneiden eingesetzt. Diese Behandlungsmethoden führen zu einer abhängig vom Verfahren zum Teil erheblichen Emission von Pilzen, Viren und Bakterien in die unmittelbare Umgebung durch das Abtragen von entsprechend verunreinigten festen Stoffen (Gewebeteile und dergleichen) und das Austreten von flüssigen Stoffen (physiologische Spülflüssigkeit, Blut, Eiter und dergleichen) und gasförmigen Stoffen (Luft, Körpergase, etc.) bei der Behandlung.

Beim Laserskalpell ist es üblich, daß auftretende Emissionen zum Teil abgesaugt werden. Die Luft wird über mechanische Umluftfilter gereinigt. Diese Filter müssen nach jeder Behandlung jedoch gewechselt werden.

Beim Fräsen und Schleifen treten die Emissionen ungehindert in den Behandlungsraum, wobei sich die größeren verunreinigten Partikel sofort in der näheren Umgebung niederschlagen, und die kleineren Partikel meist noch eine Zeit als feiner Staub bzw. feiner Nebel in der Luft des Behandlungsraums verharren und somit zur Kontaminierung und gegebenenfalls Infizierung des Behandlungspersonals führen können.

Eine vergleichbare Problematik findet sich bei chirurgischen Eingriffen mittels Wasserstrahlskalpell. Hierbei können die mit Pilzen, Viren und Bakterien kontaminierten Stoffe ebenso ungehindert in den Raum treten und sich entweder niederschlagen oder in der Luft verbleiben. Die Problematik der Sprühkontamination ist hier besonders gegeben, da die Behandlungsflüssigkeit je nach Behandlungsnotwendigkeit (Spülen, Präparieren, Schneiden) z. T. mit erheblichem Druck (bis 700 bar) aus dem Wasserstrahlskalpell tritt. Auch eine zumeist ergänzende Abdeckung der Wundrandbereiche durch entsprechende medizinische Tücher kann nur einen geringen Teil von verunreinigten Stoffen auffangen. Es bleibt jedoch die Problematik, daß bei den Verfahren die auftretenden Emissionen sowohl das Personal als auch den zu behandelnden Patienten selbst gefährden. Die Emissionen können eingeatmet werden oder schlagen sich auf die Wunden des Patienten selbst nieder. Hieraus können sich unkontrollierte Entzündungen bzw. Erkrankungen ergeben. Die bislang bekannte Vorgehensweise erfordert selbstverständlich, daß nach jeder Behandlung der Operationssaal gründlichst desinfiziert und gereinigt wird, damit das Risiko einer Übertragung von einem Patienten auf den anderen bzw. auf das behandelnde Personal minimiert wird. Dies hat wiederum während der Reinigungsarbeiten Ausfallzeiten des Operationssaales zur Folge, was zusätzlich zu den Reinigungskosten zu Ausfallkosten führt, die angesichts der hohen Stundensätze in Kliniken und Krankenhäusern erheblich sein können.

Auch eine mitunter beim Verfahren mit dem Wasserstrahlskalpell verwendete Folienabdeckung um den Patienten und den behandelnden Arzt konnte die oben genannten Probleme und Gefahren nicht beseitigen.

Ein weiterer Nachteil der bekannten Verfahren besteht darin, daß aufgrund der mangelnden Absaug- und Abhaltemöglichkeiten diese Verfahren nur in Räumen eingesetzt werden können, die eine komplette Wischdesinfektion gestatten, das heißt also in erster Linie nur in OP-Räumen. Eine oftmals angezeigte ambulante Behandlung kann somit nicht angewendet werden, wenngleich die neueren chirurgischen Methoden, wie die Lasertechnik, das Fräsen und Schleifen und das Wasserstrahlschneiden wesentliche Vorteile gegenüber den herkömmlichen Verfahren bezüglich Belastung des Patienten, Heilungsprozeß etc. aufweisen.

Der Erfindung liegt daher die Aufgabe zugrunde, ein Verfahren zur Vermeidung der Sprühkontamination bei medizinischen Eingriffen zu entwickeln, bei dem die obengenannten Nachteile vermieden werden.

Das erfindungsgemäße Verfahren löst diese Aufgabe dadurch,
a)daß um das zu behandelnde Körperteil durch eine Abschirmung ein kleiner Behandlungsraum gebildet wird,
b)daß Zugangsmöglichkeiten in das Innere des Behandlungsraumes geschaffen werden,
c)daß die Stoffe, insbesondere die gasförmigen Stoffe aus dem Inneren des Behandlungsraums abgesaugt und einer weiteren Behandlung unterzogen werden,
d)daß das Innere des Behandlungsraumes von außen einsehbar ist.

Durch die Anwendung des erfindungsgemäßen Verfahrens wird somit ein abgeschirmter Behandlungsraum um ein zu behandelndes Körperteil geschaffen, welcher dennoch zugänglich und von außen einsehbar ist. Desweiteren wird durch die Abschirmung und gleichzeitige Absaugung die Gefahr einer Kontaminierung des Patienten selbst und auch des/der behandelnden Personen weitestgehend vermieden. Insbesondere durch die zusätzliche Absaugung und Filterung kontaminierter Stoffe bzw. Luft aus dem Inneren des Behandlungsraumes erhöht sich die Sicherheit aller Beteiligten. Durch die Abschirmung auf einen vergleichsweise kleinen Behandlungsraum in unmittelbarer Umgebung des zu behandelnden Körperteils wird nur der Behandlungsraum verschmutzt und es werden damit auch die Kosten für eine Reinigung desselben erheblich reduziert. Durch die erfindungsgemäß vorgesehene ortsungebundene Anwendungsmöglichkeit des Verfahrens brauchen desweiteren die medizinischen Eingriffe nicht mehr stationär durchgeführt zu werden, sondern sind auch ambulant machbar.

Durch eine mechanische, elektrostatische oder pyrolytische Behandlung nach der Absaugung kann eine gute Reinigung der verunreinigten Stoffe erzielt werden. Besonders eine Kombination der genannten Behandlungen stellt sicher, daß die aus dem Behandlungsraum austretende und durch Pilze, Viren und Bakterien verunreinigte Luft so behandelt wird, daß Pilze, Viren oder Bakterien abgehalten und auch wirklich abgetötet werden.

Es ist zweckmäßig, wenn die verunreinigten Stoffe nach der Absaugung in Strömungsrichtung zunächst mechanisch, dann elektrostatisch gefiltert und anschließend pyrolytisch gereinigt werden. Durch diese Anordnung kann zum einen erreicht werden, daß die groben Verunreinigungen sich nur auf das mechanischen Filter absetzen und die etwas empfindlicheren nachgeschalteten Luftbehandlungsgeräte geschont werden. Außerdem muß so nur das mechanische Filter nach jeder Behandlung gereinigt werden.

Die erfindungsgemäße Vorrichtung soll sicherstellen, daß das oben beschriebene Verfahren einfach und kostengünstig angewendet werden kann. Die erfindungsgemäße Vorrichtung löst diese Aufgabe durch die Merkmale des Anspruchs 3.

Vorzugsweise besteht das Stützgerüst der Vorrichtung aus Stützstäben und einem Anschlußteil, wobei der Behälter, die Stützstäbe und das Anschlußteil der Vorrichtung voneinander lösbar sind. Auf diese Weise läßt sich die Vorrichtung leicht in ihre Bestandteile zerlegen und reinigen.

Es ist zweckmäßig, wenn die Stützstäbe abgewinkelte Rundstäbe sind. Diese sind billig in der Herstellung und lassen sich leicht montieren. Außerdem wird durch die Abwinklung eine leichte Abstützungsmöglichkeit für eine Abschirmung vorgegeben.

Gemäß einer weiteren bevorzugten Ausführungsform der erfindungsgemäßen Vorrichtung, weist der Behälter im Grundriß eine rechteckige Form auf und an seinen Querseitenwänden sind bogenförmige Mulden vorgesehen. Ein rechteckiger Behälter läßt sich leicht herstellen und an den Querseitenwänden kann eine Gliedmaße in den Behandlungsraum gebracht werden, wobei der Zugang zur zu behandelnden Gliedmaße von den Längsseiten des Behälters erleichtert wird. Die bogenförmigen Mulden gestatten eine bequeme Auflage der zu behandelnden Gliedmaße auf den Querseitenwänden.

Gemäß einer weiteren vorteilhaften Ausführungsform der Vorrichtung sind am Boden des Behälters Steckösen angebracht und das Anschlußteil besteht aus einer in ihrem Grundriß in etwa quadratischen Platte mit einer in etwa zentrischen Öffnung, einem mit der Flanschplatte verbundenen Rohrabschnitt und vier mit der Flanschplatte verbundenen Steckhülsen, wobei die Steckhülsen mit Justierschrauben versehen sind. Die Steckhülsen des Behälters und des Anschlußteils gewährleisten eine sehr einfache und schnelle Montage der Vorrichtung. Desweiteren erlauben die Justierschrauben eine einfache und sichere Justage des Stützgerüstes. Der Rohrabschnitt der Flanschplatte ermöglicht den einfachen Anschluß einer nachgeschalteten Absaug- und Filtereinrichtung.

Wenn die Absaug- und Filtereinrichtung aus einem Rohrkanal besteht, in welchem in Strömungsrichtung ein mechanisches Filter, ein elektrostatisches Filter und ein Pyrolyt angeordnet sind, so gewährleistet die Vorrichtung die Anwendung des Verfahrens in einer bevorzugten Ausführungsform.

Im folgenden wird ein Ausführungsbeispiel des erfindungsgemäßen Verfahrens anhand von Zeichnungen näher beschrieben. Dabei zeigen
- Fig. 1: den als Behälter ausgebildeten unteren Teil des Behandlungsraums in der Draufsicht;
- Fig. 2: eine Darstellung der Schnittansicht gemäß Schnittverlauf in Fig. 1;
- Fig. 3: einen Stützstab zur Abstützung einer transparenten Abschirmung in der Seitenansicht;
- Fig. 4: ein Anschlußteil von unten gesehen;
- Fig. 5: die Seitenansicht des Anschlußteils gemäß Fig. 4;
- Fig. 6: eine montierte Vorrichtung zur Anwendung des erfindungsgemäßen Verfahrens in perspektivischer Darstellung gemäß den Fig. 1 bis 5.

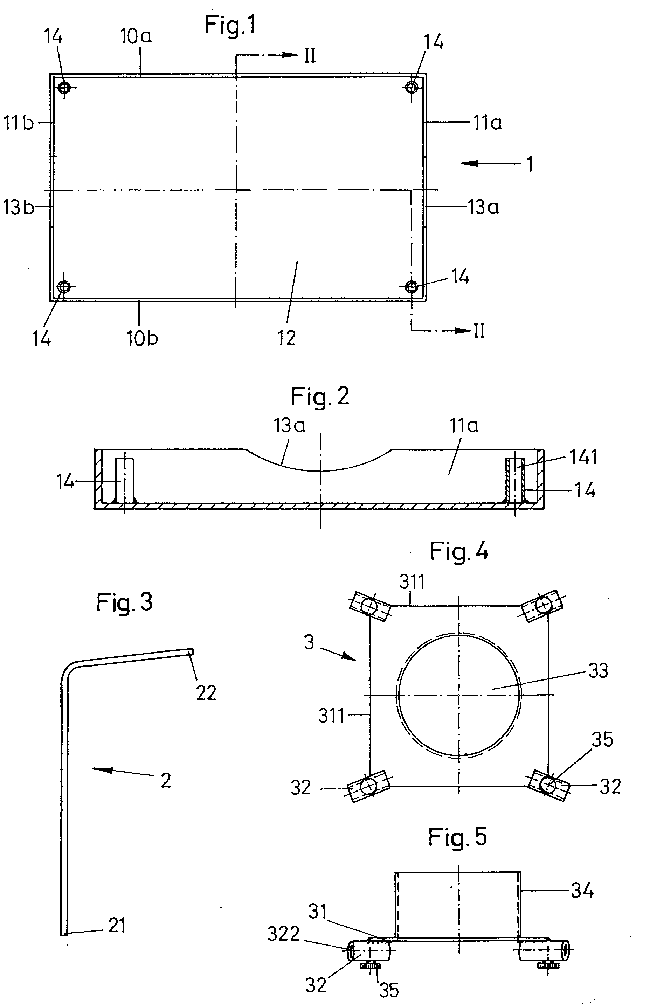

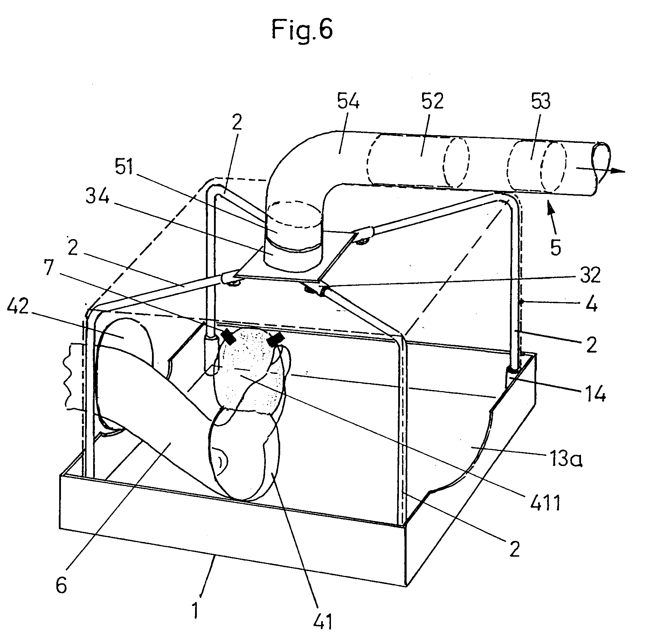

Zunächst wird auf Fig. 6 Bezug genommen. In Fig. 6 sind alle wesentlichen Vorrichtungskomponenten zur Anwendung des erfindungsgemäßen Verfahrens dargestellt. Man sieht, daß der Behandlungsraum, in dem sich die zu behandelnde Gliedmaße 6 befindet, durch einen nach oben offenen Behälter 1 und eine transparente PE-Folie 4 gebildet wird, welche von oben in den Behälter 1 eingelassen ist und sich im wesentlichen auf vier Stützstäben 2 und einem diese Stützstäbe verbindenden Anschlußteil 3 (vgl. auch Fig. 4 und 5) abstützt. Die PE-Folie 4 kann so nicht in sich zusammenfallen und kann somit einen Großteil des Behandlungsraums bilden. Der Anschlußteil 3 befindet sich in einem durch die geometrische Form der Stützstäbe 2 definierten Abstand zur oberen Öffnung des Behälters 1 und ist mit einer Absaug- und Filtereinrichtung 5 verbunden. Damit eine zu behandelnde Gliedmaße 6 in den Behandlungsraum gebracht werden kann, ist eine Öffnung 42 in die PE-Folie 4 eingebracht. Zum anderen ist in unmittelbarer Nähe der Gliedmaße 6 eine weitere Öffnung 41 in die PE-Folie 4 eingebracht, welche den Zugang einer behandelnden Person zum Innern des Behandlungsraums gestattet. Es ist weiterhin erkennbar, daß die Vorrichtung in ihren geometrischen Abmessungen nicht wesentlich größer ist als die zu behandelnde menschliche Gliedmaße 6, wodurch ein leichtes Transportieren der Vorrichtung möglich ist und damit das erfindungsgemäße Verfahren bei Bedarf leicht in unterschiedlichen Räumlichkeiten durchgeführt werden kann. Um ein Transportieren bzw. Bewegen der Vorrichtung zu erleichtern, könnte man sich ein höhenverstellbares, fahrbares Gestell vorstellen, auf das die Vorrichtung gestellt werden kann. Dies würde auch dazu beitragen, die Position der Vorrichtung so einzustellen, daß sie sowohl für den Patienten als auch für die behandelnde Person bequem ist. Es sind also die wesentlichen Komponenten der Vorrichtung dargestellt, die zur Durchführung des erfindungsgemäßen Verfahrens notwendig sind. Lediglich die behandelnde Person sowie gegebenenfalls verwendete medizinische Behandlungsgeräte, wie z. B. ein Wasserstrahlskalpell und dessen Versorgungseinrichtungen (z. B. Kompressor) sind nicht dargestellt. Desweiteren sind mögliche Elemente der Absaug- und Filtereinrichtung sowie eventuell erforderliche Versorgungsanschlüsse bzw. mobile Versorgungseinrichtungen nicht näher dargestellt.

Im folgenden sollen nun die einzelnen Komponenten der Vorrichtung, welche zur Durchführung des erfindungsgemäßen Verfahrens dienen und somit das erfindungsgemäße Verfahren näher beschrieben werden.

Fig. 1 zeigt den Behälter 1 der Vorrichtung, welcher wie bereits erwähnt einen Teil des Behandlungsraums bildet. Der nach oben offene, vergleichsweise flache Behälter weist einen im Grundriß in etwa rechteckigen Behälterboden 12, zwei Längsseitenwände 10a, 10b und zwei Querseitenwände 11a, 11b auf. Im Bereich der vier Ecken des Behälters 1 ist jeweils eine Steckhülse 14 angeschweißt. Wie Fig. 2 zeigt, sind die Steckhülsen 14 mit einer ihrer Stirnseiten mit dem Behälterboden 12 verschweißt, so daß sie sich senkrecht nach oben vom Behälterboden 12 erstrecken. Sie sind in etwa halb so hoch wie die Seitenwände des Behälters. Außerdem ist ein Abstand von einigen Millimetern zwischen den Steckhülsen 14 und den direkt angrenzenden Seitenwänden des Behälters 1 belassen, damit die PE-Folie 4 dazwischen Platz hat. Die Steckhülsen 14 weisen ferner eine Durchgangsbohrung 141 in Längsrichtung auf, welche zur Aufnahme jeweils eines Endes der Stützstäbe 2 dient. Desweiteren ist aus Fig. 1 und 2 ersichtlich, daß die Querseitenwände 11a, 11b in etwa im Bereich ihrer Mitte jeweils bogenförmige Mulden 13a, 13b aufweisen. Diese führen zu einem bequemeren Abstützen der zu behandelnden Gliedmaßen auf den Querseitenwänden. Als weitere hier nicht dargestellte Verbesserung könnte man sich an dieser Stelle auch eine schalenförmig ausgestaltete Ablagefläche für die Gliedmaßen vorstellen, was für den Patienten noch bequemer wäre. Darüber hinaus könnte man sich als hier nicht dargestellte Verbesserung der Vorrichtung einen verschließbaren Abflußflansch vorstellen, der in den Behälterboden 12 eingelassen ist. Hierdurch könnten bei Bedarf während eines medizinischen Eingriffs eventuell entstehende größere Mengen von verunreinigten Flüssigkeiten (z. B. die zum Betrieb des Wasserstrahlskalpells notwendige physiologische Flüssigkeit) auch während der Behandlung über eine ebenfalls nicht dargestellte und an den Abfluß angeschlossene Entsorgungsleitung entsorgt werden.

In Fig. 3 wird einer der vier Stützstäbe 2 von der Seite dargestellt. Der Stützstab 2 ist ein Rundstab, welcher mit seinem oberen Teil ungefähr rechtwinklig abgewinkelt ist und dort ein oberes Ende 22 aufweist. Der untere Teil des Stützstabes 2 ist in etwa so lang wie eine der Längsseitenwände 10a, 10b des Behälters 1 und in etwa doppelt so lang wie der obere, abgewinkelte Teil und weist ein unteres Ende 21 auf.

In Fig. 5 ist der Anschlußteil 3 näher dargestellt. Es ist ersichtlich, daß der Anschlußteil 3 aus einer in etwa quadratischen Flanschplatte 31, einem Rohrabschnitt 34 und vier Steckhülsen 32 besteht. Die Flanschplatte 31 weist ungefähr in der Mitte eine kreisrunde Öffnung 33 auf, an die der Rohrabschnitt 34 angeschweißt ist, wobei der Außendurchmesser des Rohrabschnitts 34 in etwa dem Durchmesser der Öffnung 33 entspricht. An den vier Ecken der Flanschplatte 31 ist jeweils eine Steckhülse 32 angeschweißt. Jede Steckhülse 32 weist dabei eine in Längsrichtung der Steckhülse verlaufende Durchgangsbohrung 322 und eine senkrecht zu dieser Durchgangsbohrung verlaufende Justierschraube 35 auf, welche in die Durchgangsbohrung hineingreifen kann. Die an der Unterseite der Flanschplatte 31 angeschweißten vier Steckhülsen 32 sind so ausgerichtet, daß die gedachten Verlängerungslinien ihrer Längsachsen sich ungefähr im Mittelpunkt der Öffnung 33 kreuzen.

Die Montage der Vorrichtung zur Anwendung des erfindungsgemäßen Verfahrens läßt sich wie folgt durchführen: Der Behälter 1 kann auf eine fahrbare oder auch feste Unterlage gestellt werden. Dann werden die Stützstäbe 2 mit dem oberen Ende 22 jeweils in die Durchgangsbohrung 322 der Steckhülsen 32 geschoben und die unteren Enden 21 der Stützstäbe 2 jeweils in die Durchgangsbohrungen 141 der Steckhülsen 14 geschoben. In dieser vormontierten Position ist das Anschlußteil 3 so ausgerichtet, daß die Flanschplatte 31 in etwa Parallel zum Behälterboden 12 und die Seiten der Flanschplatte 31 in etwa parallel zu den Seitenwänden des Behälters 1 stehen. Außerdem weist der Rohrabschnitt 34 nach oben, ist also abgewandt vom Behälterboden 12. Zur weiteren Justierung des so gebildeten Stützgerüstes werden die Stützstäbe 2 in den Steckhülsen 32 mittels der Justierschrauben 35 justiert. Nun wird eine PE-Folie von oben sackförmig über das Stützgerüst gestülpt und in den Behälter 1 so eingelassen, daß sich die PE-Folie zwischen den Steckhülsen 14 und den Seitenwänden 10a, 11a, 10b, 11b, 11b, 10a befindet. Auf diese Weise schließt die PE-Folie den Zugang zum Behälter 1 ringsum ab und bildet zusammen mit dem Behälter 1 somit den bereits erwähnten Behandlungsraum. Dadurch, daß die PE-Folie nur innen an den Seitenwänden des Behälters 1 angebracht wird, laufen von der PE-Folie herabfließende, verunreinigte Stoffe wieder in den Behälter 1 zurück und können nicht außerhalb des Behandlungsraums gelangen. Eine zusätzliche Sicherungsmöglichkeit der PE-Folie 4 könnte durch an den Seitenwänden des Behälters 1 nach innen vorstehende, kugelförmige Anformungen realisiert werden, die in vorbereitete, im unteren Bereich der PE-Folie befindliche kleine Löcher greifen könnten. Das Andrücken der Folie von innen an die Seitenwände könnte z. B. durch die Öffnung 41 erfolgen, welche ohnehin geschaffen werden muß, um der behandelnden Person den Zugang zur zu behandelnden Gliedmaße 6 zu verschaffen. Ebenso wären kleine Befestigungshaken an den Seitenwänden des Behälters 1 denkbar. Im oberen Bereich der PE-Folie 4, welche sackförmig über das Stützgerüst gestülpt wird, ist zusätzlich eine Öffnung vorgesehen, durch die der Rohrabschnitt 34 herausragen kann. Die Öffnung kann ruhig etwas kleiner als der Außendurchmesser des Rohrabschnittes 34 sein, wodurch die Abdichtwirkung an dieser Stelle noch verbessert wird.

Die Schaffung von Öffnungen 41, 42 in die PE-Folie kann relativ einfach durch Herausschneiden mit einem Messer oder einer Schere bewerkstelligt werden. Dabei ist es im Falle der für die behandelnde Person geschaffenen Zugangsöffnung 41 durchaus angebracht, nicht ein Stück vollkommen aus der PE-Folie herauszuschneiden, sondern einen PE-Folienabschnitt 411 zu belassen. Dieser kann während des Eingriffs wie in Fig. 6 gezeigt, nach oben geklappt werden und zum Beispiel mittels Pflasterstreifen 7 gesichert werden. Bei eventuell notwendiger Unterbrechung des Eingriffs kann der PE-Folienabschnitt 411 wieder heruntergeklappt und die Öffnung 41 somit zur höheren Sicherheit wieder verschlossen werden. Der Bereich der Öffnung 42, durch die die Gliedmaße 6 hindurchgesteckt worden ist, könnte zusätzlich noch mit einem Abdecktuch zur Sicherheit abgedeckt werden. Eine andere Möglichkeit zur Schaffung der Zugangsöffnungen, bestünde darin, daß die Folie bereits eine Art Sollbruchstelle an den Stellen aufweist, wo ein Zugang erfolgen soll, so daß ein Schneiden nicht mehr erforderlich ist. Eine praktische, ebenfalls nicht dargestellte Zugangsmöglichkeit für die behandelnde Person könnte darin bestehen, von vornherein nach innen gehende, handschuhformähnliche Ausstülpungen an die PE-Folie anzuformen. Hier wäre dann bei eventuellen Unterbrechungen der Behandlung keine zwischenzeitliche Desinfektion der behandelnden Person notwendig. Der Vorteil in der Verwendung einer PE-Folie liegt in der preiswerten Herstellung, der hohen Flexibilität und Reißfestigkeit und in der hohen Transparenz, welche eine gute Sicht in den Behandlungsraum bei gleichzeitiger hoher Sicherheit gewährleistet.

Zur Absaugung und Filtrierung der verunreinigten Luft ist, wie bereits erwähnt, eine Absaug- und Filtereinrichtung 5 vorgesehen. Diese Filtereinrichtung besteht aus einem Rohrkanal 54, welcher an einem Ende mittels geeigneter Mittel, wie z. B. Schellen oder Klebestreifen mit dem Rohrabschnitt 34 des Anschlußteiles 3 verbunden ist. Der Rohrkanal 54 umfaßt in Strömungsrichtung zunächst ein mechanisches Filter, welches ähnlich wie ein Naßfilter bei einer Friteuse, welches aus engvermaschtem Draht besteht, ausgeführt sein kann. Dieses filtert bereits die größeren Partikel der verunreinigten Luft heraus. Für die kleineren Partikel, wie z. B. Viren, Pilze oder Bakterien ist ein elektrostatisches Filter 52 nachgeschaltet, an welchem sich diese kleineren Partikel abscheiden. Hinter dieses elektrostatische Filter ist wiederum ein Pyrolyt 53 geschaltet, der ähnlich wie Wärmestäbe in einem Toaster widerstandsbeheizt ist und somit hohe Temperaturen erzeugen kann. Dieser dient dazu, daß sich auf dem elektrostatischen Filter abgelagerte Viren und dergleichen bei nachgeschalteten Absaugvorgängen nicht lebend in die Umwelt geblasen werden, sondern durch den stark erhitzten Pyrolyten abgetötet werden können.

Es versteht sich von selbst, daß die Komponenten der Vorrichtung auch andere geometrische Formen aufweisen können, was der Einsatzfähigkeit der Vorrichtung für das erfindungsgemäße Verfahren keinen Abbruch tut. So können z. B. die Abmaße des Behälters 1 und der Stützstäbe 2 verändert werden, wodurch sich eine Veränderung des Behandlungsraumes ergibt. Weiterhin kann die PE-Folie 4 bei Bedarf natürlich auch mit mehr als den gezeigten Öffnungen 41, 42 versehen werden, wenn die behandelnde Person z. B. von mehreren Seiten Zugang zu der zu behandelnden Gliedmaße 6 haben muß. Nach Abschluß einer Behandlung wird die Absaug- und Filtereinrichtung 5 vom Anschlußteil 3 gelöst, die PE-Folie 4 nach oben hin abgezogen und entsorgt. Der Behälter 1 und das aus den Stützstäben 2 und dem Anschlußteil 3 bestehende Stützgerüst müssen gereinigt und desinfiziert werden. Dies kann in speziell dafür vorgesehenen Einrichtungen geschehen. Hierfür ist es sehr praktisch, daß die Vorrichtung, insbesondere das Stützgerüst zerlegbar ist und somit bei der Reinigung wenig Platz verbraucht. Desgleichen muß das mechanische Filter gereinigt werden. Das nachgeschaltete elektrostatische Filter kann nach längeren Zyklen gereinigt werden.

## Patentansprüche

1. Verfahren zur Vermeidung der Sprühkontamination bei medizinischen Eingriffen, dadurch gekennzeichnet,
a) daß um das zu behandelnde Körperteil (6) durch eine Abschirmung (1, 4) ein kleiner Behandlungsraum gebildet wird,
b) daß Zugangsmöglichkeiten (41) in das Innere des Behandlungsraumes geschaffen werden,
c) daß die Stoffe, insbesondere die gasförmigen Stoffe aus dem Inneren des Behandlungsraums abgesaugt und einer weiteren Behandlung unterzogen werden,
d) daß das Innere des Behandlungsraumes von außen einsehbar ist.

2. Verfahren nach Anspruch 1, dadurch gekennzeichnet, daß die weitere Behandlung nach der Absaugung die Filterung der verunreinigten Stoffe und zwar mechanisch und/oder elektrostatisch und/oder eine pyrolytische Reinigung umfaßt.

3. Verfahren nach Anspruch 1 und 2, dadurch gekennzeichnet, daß die verunreinigten Stoffe nach der Absaugung in Strömungsrichtung zunächst mechanisch, dann elektrostatisch gefiltert und anschließend pyrolytisch gereinigt werden.

4. Vorrichtung zur Durchführung des Verfahrens zur Vermeidung der Sprühkontamination bei medizinischen Eingriffen, dadurch gekennzeichnet, daß ein nach oben hin offener Behälter (1) mit einem Stützgerüst versehen ist, geeignet zur Abstützung einer transparenten Abschirmung (4), daß die Abschirmung (4) und der Behälter (1) einen Behandlungsraum bilden, daß Zugangsöffnungen (41, 42) zum Behandlungsraum vorgesehen sind und daß eine Absaug- und Filtereinrichtung (5) vorgesehen ist.

5. Vorrichtung nach Anspruch 3, dadurch gekennzeichnet, daß das Stützgerüst aus Stützstäben (2) und Anschlußteil (3) besteht und der Behälter (1), die Stützstäbe (2) und das Anschlußteil (3) voneinander lösbar sind.

6. Vorrichtung nach Anspruch 3 und 4, dadurch gekennzeichnet, daß die Stützstäbe (2) abgewinkelte Rundstäbe sind.

7. Vorrichtung nach einem der Ansprüche 3 bis 5, dadurch gekennzeichnet, daß der Behälter (1) im Grundriß eine rechteckige Form aufweist und an seinen Querseitenwänden (11a, 11b) bogenförmige Mulden (13a, 13b) vorgesehen sind.

8. Vorrichtung nach einem der Ansprüche 3 bis 6, dadurch gekennzeichnet, daß am Boden (12) des Behälters (1) Steckhülsen (14) angebracht sind, geeignet zur Aufnahme der Stützstäbe (2).

9. Vorrichtung nach einem der Ansprüche 3 bis 7, dadurch gekennzeichnet, daß das Anschlußteil (3) aus einer in ihrem Grundriß in etwa quadratischen Platte (31) mit einer in etwa zentrischen Öffnung (33), einem mit der Flanschplatte (31) verbundenen Rohrabschnitt (34) und vier mit der Flanschplatte (31) verbundenen Steckhülsen (32), geeignet zur Aufnahme der oberen Enden (22) der Stützstäbe (2), besteht.

10. Vorrichtung nach einem der Ansprüche 3 bis 8, dadurch gekennzeichnet, daß die Steckhülsen (32) mit Justierschrauben (35) versehen sind.

11. Vorrichtung nach einem der Ansprüche 1 bis 9, dadurch gekennzeichnet, daß die Absaug- und Filtereinrichtung (5) aus einem Rohrkanal (54) besteht, in welchem in Strömungsrichtung ein magnetisches Filter (51), ein elektrostatisches Filter (52) und ein Pyrolyt (53) angeordnet sind.
